# EUROPEAN PATENT APPLICATION

(11) **EP 1 780 292 A1**
(43) Date of publication of application: **02.05.2007**
(21) Application number: 06255565.1
(22) Date of filing: 30.10.2006
(51) Int. Cl.: C12Q 1/68

(54) **Gene methylation assay controls**

(30) Priority: 31.10.2005 US 262667
(71) Applicant: Veridex, LLC, Warren, NJ 07059 (US)
(72) Inventor: Mazumder, Abhijit, Basking Ridge, NJ 07920 (US); Varde, Shobha, Hillsborough, NJ 08844 (US); Briggs, Thomas, Freemansburg, PA 18017 (US)
(74) Representative: Mercer, Christopher Paul

(57) **Abstract**

Methods, components, and kits for evaluating the effectiveness of methylation assays are presented as are methylation assays that include nucleotide sequence controls and methods of using them.

## Description

### BACKGROUND OF THE INVENTION

This invention relates to assays for methylated genes. In higher order eukaryotes DNA is methylated only at cytosines located 5' to guanosine in the CpG dinucleotide. This modification has important regulatory effects on gene expression, especially when it involves CpG rich areas (CpG islands) located in gene promoter regions. Aberrant methylation of normally unmethylated CpG islands is a frequent event in immortalized and transformed cells and has been associated with transcriptional inactivation of certain tumor suppressor genes or genes otherwise associated with the amelioration of certain human cancers.

Methylation specific PCR ("MSP") is among the best of the presently known methods for assaying gene methylation. The principle of the method is based on the differential reactivity of bisulfite with cytosine versus 5-methylcytosine. Cytosine is converted to uracil by reaction with bisulfite and alkaline desulfonation while 5-methylcytosine reacts significantly more slowly. Thus, genomic DNA containing 5-methylcytosine can be used as a substrate for PCR using primers that contain a complementary guanosine opposite the 5-methylcytosine. In contrast, genomic DNA containing cytosine and which is converted to uracil will not be primed as efficiently, due to a mismatch created by the hybridization of the guanosine opposite the uracil (created by the bisulfite reaction). In this manner, the presence of 5-methylcytosine, after quantitative PCR, will yield different Ct (cycle threshold) values than the presence of cytosine in the original genomic DNA.

The degree to which cytosines are converted to uracils is important to the success of the analysis. Ideally, all cytosines are so converted. This conversion is dependent on the degree to which the DNA is denatured making the denaturation step important as well. Improvements in these and other aspects of MSP reactions and the reagents used in them have been proposed and will continue to be developed. There is a need to have a means to compare the effect of protocol and formulation changes in these processes. Likewise, there is a need to be able to determine the efficacy of reagents used in MSP processes for quality control and other purposes. In applications involving bisulfite modification of nucleic acids, reagents are needed that would enable one to divorce the bisulfite modification step from amplification and detection methods (such as a qPCR step) in the evaluation and formulation of assays that employ such modifications. Reagents are also needed which can be used for standardization of amplification and detection processes (such as the qPCR process) which specifically report on the effects of different reaction conditions or differences in primers and probes used in the reaction.

### SUMMARY OF THE INVENTION

In one aspect of the invention, a method for evaluating the effectiveness of a methylation assay involves ligating a nucleotide sequence control into a plasmid, linearlizing the plasmid to produce a mimic of a genomic nucleic acid sample, subjecting the mimic to a methylation assay, and determining the effectiveness of the assay.

In another aspect of the invention, the nucleotide sequence control is a synthetic oligonucleotide having a methylcytosine, cytosine, or uracil in one or more known locations.

In yet another aspect of the invention, the method for evaluating the effectiveness of a methylation assay includes the use of nucleotide sequence controls having methylcytosine, cytosine, and uracil in one or more known locations.

In yet another aspect of the invention, a method for the preparation of a nucleotide sequence control involves synthesizing an oligonucleotide having methylcytosine, cytosine, or uracil in one or more known locations conducive to priming an MSP product from a linearlized plasmid.

In yet another aspect of the invention, nucleotide control sequences are synthesized oligonucleotides having methylcytosine, cytosine, or uracil in one or more known locations conducive to priming an MSP product and are formed from a linearlized plasmid.

In yet another aspect of the invention, methylation assay controls include nucleotide control sequences that are synthesized oligonucleotides having methylcytosine, cytosine, or uracil in one or more known locations conducive to priming an MSP product and are formed from a linearlized plasmid.

In yet another aspect of the invention kits for conducting methylation assays include nucleotide control sequences.

In yet another aspect of the invention, methods for conducting methylation assays include assaying nucleotide control sequences and determining whether the methylation assay was effective based upon the results of the assay of such control sequences.

### BRIEF DESCRIPTION OF THE DRAWINGS

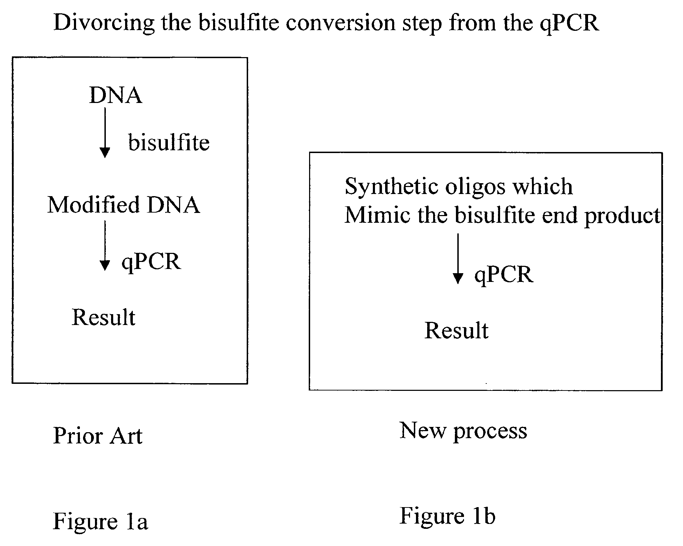

Figure 1 (a) is a schematic representation of the use of controls according to prior art.
Figure 1 (b) is a schematic representation of the use of nucleotide sequence controls according to the inventive method.

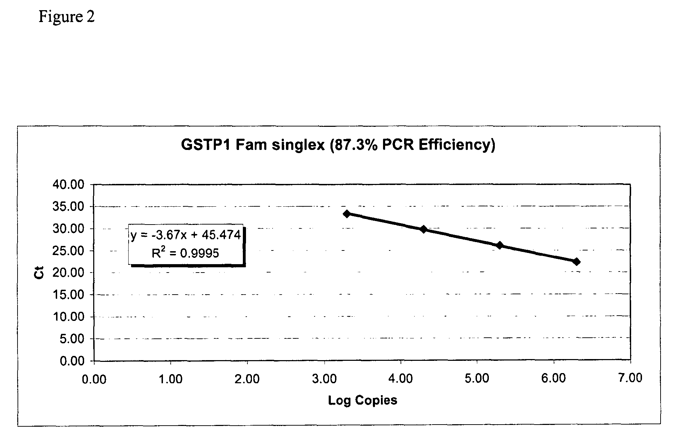

Figure 2 is a graphic representation of the results of Example 3.

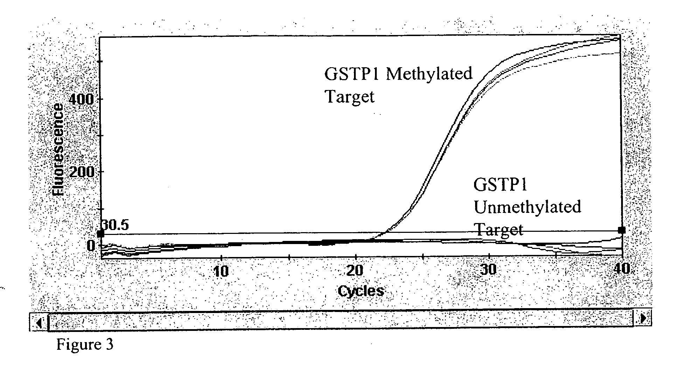

Figure 3 is a graphic representation of the results of Example 3.

### DETAILED DESCRIPTION OF THE INVENTION

### Methylation Assays

Assays for detecting hypermethylation include such techniques as MSP and restriction endonuclease analysis.

In an exemplary assay with which the inventive controls are useful, a target cell containing a Marker is contacted with a reagent that binds to the nucleic acid. Markers, in this context, are nucleotide sequences whose methylation status is significant from a diagnostic point of view. That is, knowing whether or not such sequences are hypermethylated enables a diagnosis or prognosis of a condition such as cancer or hyperplasia. The target cell component is a nucleic acid such as DNA or RNA. The reagents can include probes and primers such as PCR or MSP primers or other molecules configured to amplify and detect the target sequence. For example, the reagents can include priming sequences combined with or bonded to their own reporter segments such as those referred to as Scorpion reagents or Scorpion reporters and described in US Patents 6,326,145 and 6,270,967 to Whitcombe et. al. (incorporated herein by reference in their entirety). Though they are not precisely the same, the terms "primers" and "priming sequences" may be used in this specification to refer to molecules or portions of molecules that prime the amplification of nucleic acid sequences.

The most preferred use of the nucleotide sequence controls of this invention is in association with a methylation specific PCR reaction (MSP). That is, the controls are used to evaluate the effectiveness of such reactions, validate their operation, and provide a means of quality control for the reagents and/or kits and methods used in them.

In such MSP reactions, a nucleic acid-containing specimen is contacted with an agent that modifies unmethylated cytosine; the CpG-containing nucleic acid in the specimen is amplified by means of CpG-specific oligonucleotide primers; and the MSP products are detected as indicators of the presence of the methylated nucleic acid. The preferred modification of unmethylated cytosine is the conversion to another nucleotide that will distinguish the unmethylated from the methylated cytosine. Preferably, the agent modifies unmethylated cytosine to uracil and is sodium bisulfite, however, other agents that modify unmethylated cytosine, but not methylated cytosine can also be used. Sodium bisulfite (NaHSO₃) modification is most preferred and reacts readily with the 5,6-double bond of cytosine, but poorly with methylated cytosine. Cytosine reacts with the bisulfite ion to form a sulfonated cytosine reaction intermediate susceptible to deamination, giving rise to a sulfonated uracil. The sulfonate group can be removed under alkaline conditions, resulting in the formation of uracil. Uracil is recognized as a thymine by Taq polymerase and therefore upon PCR, the resultant product contains cytosine only at the position where 5-methylcytosine occurs in the starting template. Scorpion reporters and reagents and other detection systems similarly distinguish modified from unmodified species treated in this manner.

The primers used in the invention for amplification of a CpG-containing nucleic acid in the specimen, after modification (e.g., with bisulfite), specifically distinguish between untreated DNA, methylated, and non-methylated DNA. In MSP, primers or priming sequences for the non-methylated DNA preferably have a T in the 3' CG pair to distinguish it from the C retained in methylated DNA, and the compliment is designed for the antisense primer. MSP primers or priming sequences for non-methylated DNA usually contain relatively few Cs or Gs in the sequence since the Cs will be absent in the sense primer and the Gs absent in the antisense primer (C becomes modified to U (uracil) which is amplified as T (thymidine) in the amplification product).

Primers that are useful in the methylation process are oligonucleotides of sufficient length and appropriate sequence so as to provide specific initiation of polymerization on a significant number of nucleic acids in the polymorphic locus. When exposed to probes or reporters, the sequences that are amplified by the primers reveal methylation status and thus diagnostic information.

The primers used in the methylation assays are most preferably eight or more deoxyribonucleotides or ribonucleotides capable of initiating synthesis of a primer extension product, which is substantially complementary to a polymorphic locus strand. Environmental conditions conducive to synthesis include the presence of nucleoside triphosphates and an agent for polymerization, such as DNA polymerase, and a suitable temperature and pH. The priming segment of the primer or priming sequence is preferably single stranded for maximum efficiency in amplification, but may be double stranded. If double stranded, the primer is first treated to separate its strands before being used to prepare extension products. The primer must be sufficiently long to prime the synthesis of extension products in the presence of the inducing agent for polymerization. The exact length of primer will depend on factors such as temperature, buffer, and nucleotide composition. The oligonucleotide primers most preferably contain about 12-20 nucleotides although they may contain more or fewer nucleotides, preferably according to well-known design guidelines or rules.

Primers are designed to be substantially complementary to each strand of the genomic locus to be amplified and include the appropriate G or C nucleotides as discussed above. This means that the primers must be sufficiently complementary to hybridize with their respective strands under conditions that allow the agent for polymerization to perform. In other words, the primers should have sufficient complementarity with the 5' and 3' flanking sequence(s) to hybridize and permit amplification of the genomic locus.

The primers employed in the methylation assays produce greater quantities of target locus relative to the number of reaction steps involved, most preferably exponentially greater quantities of the target locus. Typically, one primer is complementary to the negative (-) strand of the locus and the other is complementary to the positive (+) strand. Annealing the primers to denatured nucleic acid followed by extension with an enzyme, such as the large fragment of DNA Polymerase I (Klenow) and nucleotides, results in newly synthesized + and - strands containing the target locus sequence. The product of the chain reaction is a discrete nucleic acid duplex with termini corresponding to the ends of the specific primers employed.

Any nucleic acid specimen, in purified or non-purified form, can be utilized as the starting nucleic acid or acids, provided it contains, or is suspected of containing, the specific nucleic acid sequence containing the target locus (e.g., CpG). Thus, the process may employ, for example, DNA or RNA, including messenger RNA. The DNA or RNA may be single stranded or double stranded. In the event that RNA is to be used as a template, enzymes, and/or conditions optimal for reverse transcribing the template to DNA would be utilized. In addition, a DNA-RNA hybrid containing one strand of each may be utilized. A mixture of nucleic acids may also be employed, or the nucleic acids produced in a previous amplification reaction herein, using the same or different primers may be so utilized. The specific nucleic acid sequence to be amplified, i.e., the target locus, may be a fraction of a larger molecule or can be present initially as a discrete molecule so that the specific sequence constitutes the entire nucleic acid.

The nucleic acid-containing specimen used for detection of methylated CpG may be from any source such as tissue (particularly, prostate tissue and lymphatic tissue), blood, lymph, urine, and ejaculate and may be extracted by a variety of techniques such as that described by Maniatis, et al. (Molecular Cloning: A Laboratory Manual, Cold Spring Harbor, N.Y., pp 280, 281, 1982).

If the extracted sample is impure, it may be treated before amplification with an amount of a reagent effective to open the cells, fluids, tissues, or animal cell membranes of the sample, and to expose and/or separate the strand(s) of the nucleic acid(s). This lysing and nucleic acid denaturing step to expose and separate the strands will allow amplification to occur much more readily.

Where the target nucleic acid sequence of the sample contains two strands, it is necessary to separate the strands of the nucleic acid before it can be used as the template. Strand separation can be effected either as a separate step or simultaneously with the synthesis of the primer extension products. This strand separation can be accomplished using various suitable denaturing conditions, including physical, chemical or enzymatic means. One physical method of separating nucleic acid strands involves heating the nucleic acid until it is denatured. Typical heat denaturation may involve temperatures ranging from about 80 to 105°C for up to 10 minutes. Strand separation may also be induced by an enzyme from the class of enzymes known as helicases or by the enzyme RecA, which has helicase activity, and in the presence of riboATP, is known to denature DNA. Reaction conditions that are suitable for strand separation of nucleic acids using helicases are described by Kuhn Hoffmann-Berling (CSH-Quantitative Biology, 43:63, 1978). Techniques for using RecA are reviewed in C. Radding (Ann. Rev. Genetics, 16:405-437, 1982). Refinements of these techniques are now also well known.

When complementary strands of nucleic acid or acids are separated, regardless of whether the nucleic acid was originally double or single stranded, the separated strands are ready to be used as a template for the synthesis of additional nucleic acid strands. This synthesis is performed under conditions allowing hybridization of primers to templates to occur. Generally synthesis occurs in a buffered aqueous solution, preferably at a pH of 7-9, most preferably about 8. A molar excess (for genomic nucleic acid, usually about 10⁸: 1, primer:template) of the two oligonucleotide primers is preferably added to the buffer containing the separated template strands. The amount of complementary strand may not be known if the process of the invention is used for diagnostic applications, so the amount of primer relative to the amount of complementary strand cannot always be determined with certainty. As a practical matter, however, the amount of primer added will generally be in molar excess over the amount of complementary strand (template) when the sequence to be amplified is contained in a mixture of complicated long-chain nucleic acid strands. A large molar excess is preferred to improve the efficiency of the process.

The deoxyribonucleoside triphosphates dATP, dCTP, dGTP, and dTTP are added to the synthesis mixture, either separately or together with the primers, in adequate amounts and the resulting solution is heated to about 90-100°C for up to 10 minutes, preferably from 1 to 4 minutes. After this heating period, the solution is allowed to cool to room temperature, which is preferable for the primer hybridization. To the cooled mixture is added an appropriate agent for effecting the primer extension reaction (the "agent for polymerization"), and the reaction is allowed to occur under conditions known in the art. The agent for polymerization may also be added together with the other reagents if it is heat stable. This synthesis (or amplification) reaction may occur at room temperature up to a temperature at which the agent for polymerization no longer functions.

The agent for polymerization may be any compound or system that will function to accomplish the synthesis of primer extension products, preferably enzymes. Suitable enzymes for this purpose include, for example, E. coli DNA polymerase 1, Klenow fragment of E. coli DNA polymerase I, T4 DNA polymerase, other available DNA polymerases, polymerase muteins, reverse transcriptase, and other enzymes, including heat-stable enzymes (e.g., those enzymes which perform primer extension after being subjected to temperatures sufficiently elevated to cause denaturating). A preferred agent is Taq polymerase. Suitable enzymes will facilitate combination of the nucleotides in the proper manner to form the primer extension products complementary to each locus nucleic acid strand. Generally, the synthesis will be initiated at the 3' end of each primer and proceed in the 5' direction along the template strand, until synthesis terminates, producing molecules of different lengths. There may be agents for polymerization, however, which initiate synthesis at the 5' end and proceed in the other direction, using the same process as described above.

Alternative methods of amplification can also be employed as long as the methylated and non-methylated loci is amplified sufficiently to be detected in the alternative means. Assays based on restriction endonuclease activity are also sensitive methods of detecting methylation patterns. They involve combining the use of methylation-sensitive enzymes and the polymerase chain reaction (PCR). After digestion of DNA with the enzyme, PCR will amplify from primers flanking the restriction site only if DNA cleavage was prevented by methylation.

The amplified products are preferably identified as methylated or non-methylated with a probe or reporter specific to the product as described in US Patent 4,683,195 to Mullis et. al.*,* incorporated herein by reference in its entirety. Advances in the field of probes and reporters for detecting polynucleotides are well known to those skilled in the art. Optionally, the methylation pattern of the nucleic acid can be confirmed by other techniques such as restriction enzyme digestion and Southern blot analysis. Examples of methylation sensitive restriction endonucleases which can be used to detect 5'CpG methylation include SmaI, SacII, EagI, MspI, HpaII, BstUI and BssHII.

Determining a methylation ratio is desirable in many methylation assays. This can be done by establishing a ratio between the amount of amplified methylated species of Marker attained and the amount of amplified reference Marker or non-methylated Marker region amplified. This is best done using quantitive realtime PCR. Ratios above an established or predetermined cutoff or threshold are considered hypermethylated and indicative of having a proliferative disorder such as cancer (prostate cancer in the case of GSTP1). Cutoffs are established according to known methods in which such methods are used for at least two sets of samples: those with known diseased conditions and those with known normal conditions. The reference Markers of the invention can also be used as internal controls. The reference Marker is preferably a gene that is constitutively expressed in the cells of the samples such as beta-Actin.

The inventive methods and kits can include steps and reagents for multiplexing. That is, more than one Marker can be assayed at a time.

### Nucleotide Sequence Controls

The nucleotide sequence controls of the invention are oligonucleotides that correspond to the amplicons, targets, or Markers of a methylation assay. The degree to which they so correspond depends, in part, upon the purpose they will serve. In instances in which the controls are used for quality control purposes this correspondence is very close. In such applications, it is most preferred that the controls are almost identical to the targets or amplicons. In instances in which the controls are to be used as internal controls for assay kits at least a portion of the control overlaps with the actual Marker, target or amplicon of the assay. Preferably, such assays employ the controls in a separate vessel.

In applications such as the evaluation of proposed modifications to assay protocols, the control sequences need only be similar to the sequences near the target or amplicon but capable of participating in the evaluated reaction in similar fashion. It is most preferred that the controls be nearly identical to the targets or amplicons.

The controls will have a 5-methylcytosine, an unmodified cytosine, or a uracil base in a location significant to the methylation assay scheme. Preferably, each control has three such bases. Preferably, they are unlabeled and can therefore be used as the target in a qPCR. Their placement in a location significant to the methylation assay scheme means that their presence or absence in those locations permits the interrogation of the course of the reaction. Where the controls are used to evaluate or formulate assay conditions or protocols, all three types of controls are preferably used (i.e., at least one with a 5-methyl group positioned in a significant location, at least one with an unmodified cytosine positioned in a significant location, and at least one with a uracil positioned in a significant location).

In one aspect of the invention, the nucleotide sequence controls of the invention are preferably constructed from linearlized plasmid sequences according to methods common to molecular biology.

A plasmid used to construct the nucleotide sequence control can advantageously contain all of the necessary components found in any bacterial plasmid used in the field of molecular biology. For example, an origin of replication that would permit it to replicate within host bacteria may be included. Further, a suitable plasmid may contain a multiple cloning site to facilitate the cloning of the target sequence to be used in the nucleotide sequence control. Other necessary or useful plasmid components may also be included in a plasmid used to construct a nucleotide sequence control.

Any plasmid capable of replication in bacteria and suitable for molecular biological manipulation may serve. The multiple cloning region of the control plasmid can be cut with restriction endonucleases that correspond to the sites on the fragment constructed to serve as the control. The restriction enzyme digestion creates a linear plasmid that accepts the exogenous nucleic acid sample that is the control sequence. The digested control plasmid is then isolated from the digestion reaction using standard techniques. Those techniques include but are not limited to the use of glass beads, various column matrices, gel isolation, etc.

The purified cleaved plasmid is then mixed with the purified fragment and the DNA molecules are ligated together using standard techniques known in the art. A DNA ligase is used to close the phosphate backbone of the newly formed internal control plasmid.

Oligonucleotides are commonly synthesized on solid supports by the phosphoramidite method (U.S. Pat. Nos. 4,415,732; 4,973,679; 4,458,066; Beaucage, S. and Iyer, R. (1992) Tetrahedron 48:2223-2311) using commercially available phosphoramidite nucleosides, supports e.g. silica, controlled-pore-glass (U.S. Pat. No. 4,458,066) and polystyrene (U.S. Pat. Nos. 5,047,524 and 5,262,530) and automated synthesizers (Models 392, 394, 3948 DNA/RNA Synthesizers, Applied Biosystems).

### Methods of Using Nucleotide Sequence Controls in Methylation Assays

In amplification assays one typically uses a positive control sample to provide a check to insure the functionality of the assay's reagents. If there is no signal in the experimental well but a signal in the control well, one usually concludes that there is no target sequence in the experimental well, since the result from the control well indicates that the assay's reagents are functional. The negative control sample provides a means to determine the background signal.

In the methylation assays of this invention, the PCR reaction is usually conducted after modification of the sample with, for example, bisulfite. The amplification of methylated and un-methylated species is preferably conducted in parallel. Thus,a good control strategy should test the amplification of modified species and unmodified species and, preferably, amplification of species that should have been modified but were not.

This is accomplished by formulating three different sets of controls with particular bases at positions of significance to the amplification of a gene sequence whose hypermethylation has diagnostic or prognostic significance. Preferably, one such control is made with three 5-methylcytosines; the second oligonucleotide has three cytosines; and the third with three uracil nucleotides. Bisulfite treatment is then performed (or not performed as a control) on aliquots of the sample as well as each of the three control sequences. Quantitative PCR is then performed, e.g., using primers designed to hybridize to unmodified cytosines, and the PCR profiles are compared. If the assay is properly functioning, then the control with the 5-methylcytosines exhibits the lowest Ct (cycle threshold) values while the control containing the uracils would exhibit the highest Ct values. Furthermore, the plasmid containing the cytosines would exhibit Ct values equal to or in between these two extremes, depending on which primer is used to query which cytosine (of the original three) and what percent conversion was obtained in the bisulfite reaction.

### Methods for Evaluating Methylation Assays

In one preferred and exemplary embodiment of the invention four 100-base oligonucleotides (SEQ ID 1, SEQ ID 2, SEQ ID 3, SEQ ID4) are used to evaluate a methylation assay based on the hypermethylation of GSTP1 (SEQ ID No. 7). Here, SEQ Ids 1 and 3 are hybridized to each other and SEQ Ids 2 and 4 are hybridized to each other to form two separate duplexes. SEQ ID 1 represents 100 bases of the top strand of the GSTP1 gene after bisulfite modification, (if all of the cytosines which preceded a guanosine were methylated). All of the remaining cytosines were replaced by uracils to generate the end product of the bisulfite reaction (if 100% of cytosines are converted to uracils). SEQ ID2 represents 100 bases of the top strand of the GSTP1 gene after bisulfite modification (if none of the cytosines which preceded a guanosine were methylated). Thus, all of the cytosines were replaced by uracils in this oligonucleotide. SEQ ID3 represents 100 bases of the bottom strand of the GSTP1 gene after bisulfite modification (if all of the cytosines which preceded a guanosine were methylated). All of the remaining cytosines were replaced by uracils to generate the end product of the bisulfite reaction (if 100% of the cytosines are converted to uracils). SEQ ID 4 represents 100 bases of the bottom strand of the GSTP1 gene after bisulfite modification (if none of the cytosines which preceded a guanosine were methylated). Thus, all of the cytosines were replaced by uracils in this oligonucleotide. Similarly, SEQ ID 5 and SEQ ID 6 can be hybridized to each other. SEQ ID 5 represents 100 bases of the top strand of the beta-actin gene after bisulfite modification (if none of the cytosines were methylated). All of the cytosines were replaced by uracils to generate the end product of the bisulfite reaction (if 100% of the cytosines are converted to uracils). SEQ ID 6 represents 100 bases of the bottom strand of the beta actin gene after bisulfite modification (if none of the cytosines were methylated). Thus, all of the cytosines were replaced by uracils in this oligonucleotide.

Because these products represent the end-product of bisulfite conversion, the use of each of these duplexes will measure only effects of the qPCR. Thus, the duplexes are useful for:
1) determining conditions which show optimal performance (e.g.., sensitivity) for detection of methylated sequences and not unmethylated sequences
2) determining reproducibility of multiple reagent lots (enzyme mixes, probe or primer syntheses, etc.)
3) standardization or quantitation of PCR reactions
4) serving as positive and negative controls for the PCR

Notably, only the effects of reagents and processes downstream of the bisulfite reaction are measured in the approach described above. In this manner, one can divorce any confounding effects from incomplete or inconsistent (complete at some cytosines but partial at others) bisulfite conversion. This same approach can be taken with controls for other methylation assays. Indeed, it can be used with other assays in which a target is modified in a step distinct from its amplification and/or detection.

### Kits:

The kits of the invention can be configured with a variety of components provided that they all contain at least one nucleotide sequence control according to the invention. In one embodiment, the kit includes reagents for amplifying and detecting hypermethylated Marker segments. Optionally, the kit includes sample preparation reagents and /or articles (e.g., tubes) to extract nucleic acids from samples.

In a preferred kit, reagents necessary for one-tube MSP are included such as, a corresponding PCR primer set, a thermostable DNA polymerase, such as Taq polymerase, and a suitable detection reagent(s) such as hydrolysis probe or molecular beacon. In optionally preferred kits, detection reagents are Scorpion reporters or reagents. A single dye primer or a fluorescent dye specific to double-stranded DNA such as ethidium bromide can also be used. The primers are preferably in quantities that yield high concentrations. Additional materials in the kit may include: suitable reaction tubes or vials, a barrier composition, typically a wax bead, optionally including magnesium; necessary buffers and reagents such as dNTPs; control nucleic acid (s) and/or any additional buffers, compounds, co-factors, ionic constituents, proteins and enzymes, polymers, and the like that may be used in MSP reactions. Optionally, the kits include nucleic acid extraction reagents and materials.

### EXAMPLES

### Example 1 : Preparation of Nucleotide Sequence Controls

Synthetic 100 base oligonucleotides were synthesized using standard phosphoramidite chemistry by TriLink Biotechnologies (San Diego, CA). 5-methyl-cytidine and uridine phosphoramidites were synthesized at the desired locations. Oligonucleotides were purified using polyacrylamide gel electrophoresis. Oligonucleotide concentration was determined by spectrophotometric measurement at 260nm wavelength. SEQ ID1 and SEQ ID3 were hybridized to each other and diluted serially for use in the qPCR assays. SEQ ID2 and SEQ ID4 were hybridized to each other and diluted serially for use in the qPCR assays.

### Example 2: Use of Controls in an MSP Reaction

Synthetic controls were amplified in a 25 µl reaction containing the following components: 67mM Tris pH 8.8,16.6mM (NH₄)₂SO₄, 6.7mM MgCl₂, 10mM beta mercaptoethanol; 1.25mM each dATP, dCTP, dGTP, dTTP, 1 U Hot start Taq DNA Ploymerase, 250 nM Scorpion probe, and 250 nM reverse or forward primer (depending on scorpion design). The samples were then tested in a quantitative real-time PCR assay on a Cepheid SmartCycler^{®} PCR instrument. The PCR conditions used were: 95°C for 60 sec; then 40 cycles of 95°C for 30sec, 59°C for 30 sec, and a final extension at 72°C for 5 min. Optical data was collected at 59°C for every cycle. The results, shown below, demonstrate that, the Scorpion assays can detect methylated GSTP1 and that the beta-actin assays, designed to measure unmethylated beta-actin, do so. The results also demonstrate the reproducibility of the assays.

| Gene | Ct value | SD | Endpoint fluorescence | SD |
|---|---|---|---|---|
| GSTP1 | 27.1 | 0.2 | 548 | 28 |
| β-actin | 28.5 | 0.2 | 218 | 14 |

### Example 3:Use of the Controls to Validate an Assay Protocol

In order to validate that 1) the GSTP1 Scorpion assay would detect only methylated DNA after treatment with bisulfite and not unmethylated DNA, that 2) the GSTP1 Scorpion assay was a highly sensitive assay (i.e., very few copy numbers could be detected), and 3) that the assay was highly linear, we generated standard curves using serial dilutions of each of the oligonucleotide duplexes. Results are shown in Tables 1 and 2.

**Table 1**

| **Singlex GSTP1 Meth Synthetic control** | | | | | | | | | | | | | |
|---|---|---|---|---|---|---|---|---|---|---|---|---|---|
| **Detector** | **Task** | **Quantity** | **Log copies** | **Ct 1** | **Ct 2** | **Ct 3** | **Ct 4** | **Average Ct** | **Delta Ct** | **E.F 1** | **E.F 2** | **E.F 3** | **E.F 4** |
| Gst-Pi M | Standard | 2E+06 | 6.30 | **22.20** | **22.30** | **22.20** | **22.30** | 22.25 | 0.10 | **558** | **567** | **519** | **556** |
| Gst-Pi M | Standard | 2E+05 | 5.30 | **26.50** | **25.90** | **26.10** | **26.00** | 26.13 | 0.50 | **439** | **452** | **545** | **532** |
| Gst-Pi M | Standard | 20000 | 4.30 | **30.20** | **29.80** | **29.50** | **29.60** | 29.78 | 0.60 | **412** | **468** | **526** | **409** |
| Gst-Pi M | Standard | 2000 | 3.30 | 34.10 | **33.20** | **33.50** | **33.10** | 33.27 | 0.10 | **165** | **282** | **336** | **356** |
| Gst-Pi M | Standard | 200 | 2.30 | 38.90 | 39.00 | 36.70 | 37.20 | 37.95 | *1.70* | 52 | 49 | 150 | 118 |
| Gst-Pi M | Standard | 20 | 1.30 | *0.00* | *0.00* | *0.00* | *0.00* | 0.00 | *0.00* | -4 | 17 | -15 | -27 |
| Gst-Pi M | NTC | | | 0.00 | 0.00 | 0.00 | 0.00 | 0.00 | 0.00 | 0 | 0 | -27 | 8 |

**Table 2**

| **Singlex GSTP1 Unmethylated Synthetic control** | | | | | | | | | | | | | |
|---|---|---|---|---|---|---|---|---|---|---|---|---|---|
| **Detector** | **Task** | **Quantity** | **Log copies** | **Ct 1** | **Ct 2** | **Ct 3** | **Ct 4** | **Average Ct** | **Delta Ct** | **E.F 1** | **E.F 2** | **E.F 3** | **E.F 4** |
| Gst-Pi U | Standard | 2E+06 | 6.30 | 0.00 | 0.00 | 0.00 | 0.00 | 0.00 | 0.00 | -36 | -15 | -19 | 13 |
| Gst-Pi U | Standard | 2E+05 | 5.30 | 0.00 | 0.00 | 0.00 | 0.00 | 0.00 | 0.00 | -36 | -27 | -13 | 3 |
| Gst-Pi U | Standard | 20000 | 4.30 | 0.00 | 0.00 | 0.00 | 0.00 | 0.00 | 0.00 | -23 | -36 | -37 | -47 |
| Gst-Pi U | Standard | 2000 | 3.30 | 0.00 | 0.00 | 0.00 | 0.00 | 0.00 | 0.00 | -51 | -24 | 1 | -25 |
| Gst-Pi U | Standard | 200 | 2.30 | 0.00 | 0.00 | 0.00 | 0.00 | 0.00 | 0.00 | -33 | -9 | -24 | -10 |
| | | | | | | | | | | | | -38 | |
| Gst-Pi U | Standard | 20 | 1.30 | 0.00 | 0.00 | 0.00 | 0.00 | 0.00 | 0.00 | -71 | -56 | | -25 |
| Gst-Pi U | NTC | | | 0.00 | 0.00 | 0.00 | 0.00 | 0.00 | 0.00 | 0 | 0 | -58 | -34 |

### Example 4 (Comparative and Prophetic)

Using the best prior art to determine the performance of a reporter in the Methylation-Specific PCR would result in the investigator having to sort out performance issues due solely to the reporter versus performance due to the reporter and/or incomplete or partial conversion in the bisulfite reaction. Ordinarily, a design of experiment (DOE) type investigation would be constructed to investigate several PCR parameters (e.g., buffer conditions, enzyme concentration, primer and probe concentration). However, the success of these experiments relies on the assumption that the previous bisulfite conversion has been successful.

In this invention, synthetic targets which mimic the end product of the bisulfite conversion are used in the DOE and therefore only effects due to the subsequent PCR conditions will be reported.

## Claims

1. A method of generating nucleotide sequence controls comprising: a) synthesis of oligonucleotides containing a 5-methylcytosine adjacent to a guanosine or a uracil in place of a cytosine, b) annealing the oligonucleotides to form a synthetic duplex, c) using the synthetic duplex under the reaction conditions of an assay for modified nucleic acids, and d) evaluating the performance of the assay.

2. The method according to claim 1 wherein the synthesis of oligonucleotides is conducted in the presence of restriction and/or ligation enzymes to clone or ligate such controls into a plasmid or other DNA.

3. The method according to claim 1 wherein the synthesis of oligonucleotides is automated.

4. The method according to claim 2 wherein the plasmid is circularized or linear.

5. The method of claim 1 wherein the nucleotide sequence controls are used for quality control testing of an assay involving the modification of a nucleic acid.

6. The method of claim 5 wherein the quality control testing includes the standardization of reagent batches, standardization of assay performance for different instruments, the standardization of performance for different formats, the standardization of performance of different sites, and the standardization of performance for different operators of the assay.

7. The method of claim 6 wherein the standardization of different formats includes the use of microtiter plates, tubes, wells, capillary devices, tubing, and columns.

8. The method according to claim 1 wherein the synthetic oligonucleotide is greater than or equal to 100 bases in length.

9. The method of claim 1 wherein multiple duplexes are used.

10. The method of claim 1 wherein the synthetic duplexes are used in a quantitative PCR or in an endpoint PCR.

11. The method of claim 1 wherein the synthetic duplexes are used as positive controls for the methylation specific PCR when patient samples are used or when the synthetic duplexes are used to generate a standard curve for quantitation purposes.

12. The method of claim 1 wherein the synthetic duplexes contain no 5-methylcytosines.

13. The method of claim 12 wherein the duplexes are used to evaluate the specificity of an msPCR assay.

14. The method of claim 1 wherein mixtures of duplexes are generated having only 5-methylcytosines and uracils.

15. The method of claim 14 used to evaluate the background of unmethylated DNA in a DNA methylation assay.

16. The method of claim 1 wherein a mixture of two different types of duplexes is generated and they are used to estimate or determine the extent of bisulfite conversion.

17. A composition comprising a member of the group consisting of Seq. ID No. 1-6.

18. A kit comprising a composition of claim 17.

19. A kit for conducting an assay for a modified nucleic acid comprising nucleotide sequence controls.

20. The kit of claim 19 comprising more than one nucleotide control sequence.

21. The kit of claim 20 wherein the nucleotide control sequences are provided for more than one gene.

22. The kit of claim 19 further comprising instructions.

23. The kit of claim 19 further comprising reagents for amplifying and detecting the presence of a methylated gene.

24. The kit of claim 19 further comprising reagents for amplifying and detecting the presence of constitutively expressed genes.

25. A method for assaying modified nucleic acids comprising,
a) reacting a nucleic acid suspected of having a modification with a reagent to form a product,
b) amplifying the product of step a),
c) subjecting nucleic acid sequence controls to amplification conditions, and
d) detecting the presence of the products of steps b) and c).

26. The method of claim 25 wherein the modified nucleic acid is one that is methylated.

27. The method of claim 25 wherein the reagent of step a) is bisulfite or a composition that results in the presence of bisulfite.

28. The method of claim 26 further comprising establishing a methylation ratio and determining whether the methylation ratio exceeds a cutoff value.

29. The method of claim 1 for use in methylation specific PCR.

30. A method of generating bisulfite-end product controls which can be used for optimizing PCR conditions in a methylation-specific PCR assay, divorced from the bisulfite conversion step, the methods comprising: a) automated DNA synthesis of 100 base oligonucleotides containing a 5-methylcytosine adjacent to a guanosine or a uracil in place of a cytosine, representing the end product of a complete bisulfite conversion, b) annealing of the synthetic single stranded oligonucleotides to form a synthetic duplex, c) use of the synthetic duplex in conjunction with PCR thermocycling conditions, buffers, enzymes, and a reporter system, preferably, Scorpions, to optimize a methylation specific PCR assay.


SEQUENCE LISTING

<110> VERIDEX, LLC
MAZUMDER, Abhijit
VARDE, Shobha
BRIGGS, Thomas

<120> GENE METHYLATION ASSAY CONTROLS

<130> P045263EP

<150> US 11/262667
<151> 2005-10-28

<160> 7

<170> PatentIn version 3.3

<210> 1
<211> 100
<212> DNA
<213> Artificial

<220>
<223> oligonucleotide

<220>
<221> modified_base
<222> 1, 4, 8, 18, 22, 25, 37, 40, 48, 50, 68, 72, 75, 78, 96
<223> m5c

<400> 1
cgacguucgg ggtguagcgg ucgucggggu tggggucggc gggagtucgc gggauuutuu 60

agaagagcgg ucggcgucgt gautuaguau tggggcggag 100

<210> 2
<211> 100
<212> DNA
<213> Artificial

<220>
<223> oligonucleotide

<400> 2
ugauguuugg ggtguagugg uuguuggggu tgggguuggu gggagtuugu gggauuutuu 60

agaagagugg uugguguugt gautuaguau tgggguggag 100

<210> 3
<211> 100
<212> DNA
<213> Artificial

<220>
<223> oligonucleotide

<220>

<221>  modified_base
<222>  4, 22, 25, 28, 32, 50, 52, 60, 63, 75, 78, 82, 92, 96, 99
<223>  m5c

<400>  3
utucguuuua gtgutgagtu acggcgucgg ucgututtut ggagggtuuc gcggautuuc     60

gucgguuuua guuucggcgg ucgutguauu ucgggcgtcg                          100


<210>  4
<211>  100
<212>  DNA
<213>  Artificial

<220>
<223>  oligonucleotide

<400>  4
utuuguuuua gtgutgagtu augguguugg uugututtut ggagggtuuu guggautuuu     60

guugguuuua guuuuggugg uugutguauu uugggugtug                          100


<210>  5
<211>  100
<212>  DNA
<213>  Artificial

<220>
<223>  oligonucleotide

<400>  5
ggagtataua ggutggggaa gtttguuutt gugtggggtg gtgatggagg aggutuagua     60

agtuttutgg autgtgaauu tgtgtutguu autgtgtgut                          100


<210>  6
<211>  100
<212>  DNA
<213>  Artificial

<220>
<223>  oligonucleotide

<400>  6
aauauauaat aauaaauaua aattuauaat uuaaaaaaut tautaaauut uutuuatuau     60

uauuuuauau aaaaacaaau ttuuuuaauu tatatautuu                          100


<210>  7
<211>  4260
<212>  DNA
<213>  Homo sapiens


<300>
<301>  Morrow,C.S., Cowan,K.H. and Goldsmith,M.E.
<302>  Structure of the human genomic glutathione S-transferase-pi gene

<303>  Gene
<304>  75
<305>  1
<306>  3-11
<307>  1989-01-30
<308>  gi|341173|gb|M24485.1|HUMGSTP1G
<309>  1994-12-05

<400>  7
aacaagagat caatatctag aataaatgga gatctgcaaa tcaacagaaa gtaggcagca      60

aagccaaaga aaatagccta aggcacagcc actaaaagga acgtgatcat gtcctttgca     120

gggacatggg tggagctgga agccgttagc ctcagcaaac tcacacagga acagaaaacc     180

agcgagaccg catggtctca cttataagtg ggagctgaac aatgagaaca catggtcaca     240

tggcggcgat caacacacac tggtgcctgt tgagcggggt gctggggagg gagagtacca     300

ggaagaatag ctaagggata ctgggcttaa tacctgggtg atgggatgat ctgtacagca     360

aaccatcatg gcgcacacac ctatgtaaca aacctgcaca tcctgcacat gtaccccaga     420

acttcaaata aaagttggac ggccaggcgt ggtggctcac gcctgtaatc ccagcacttt     480

gggaagccga ggcgtgcaga tcacctaagg tcaggagttc gagaccagcc cggccaacat     540

ggtgaaaccc cgtctctact aaaaatacaa aaatcagcca gatgtggcac gcacctataa     600

ttccacctac tcgggaggct gaagcagaat tgcttgaacc cgagaggcgg aggttgcagt     660

gagccgccga gatcgcgcca ctgcactcca gcctgggcca cagcgtgaga ctacgtcata     720

aaataaaata aaataacaca aaataaaata aaataaaata aaataaaata aaataataaa     780

ataaaataaa ataaaataaa ataaaataaa ataaagcaat ttcctttcct ctaagcggcc     840

tccacccctc tcccctgccc tgtgaagcgg gtgtgcaagc tccgggatcg cagcggtctt     900

agggaatttc cccccgcgat gtcccggcgc gccagttcgc tgcgcacact tcgctgcggt     960

cctcttcctg ctgtctgttt actccctagg ccccgctggg gacctgggaa agagggaaag    1020

gcttccccgg ccagctgcgc ggcgactccg gggactccag ggcgcccctc tgcggccgac    1080

gcccggggtg cagcggccgc cggggctggg gccggcggga gtccgcggga ccctccagaa    1140

gagcggccgg cgccgtgact cagcactggg gcggagcggg gcgggaccac ccttataagg    1200

ctcggaggcc gcgaggcctt cgctggagtt tcgccgccgc agtcttcgcc accagtgagt    1260

acgcgcggcc cgctccccgg ggatggggct cagagctccc agcatggggc caacccgcag    1320

catcaggccc gggctcccgg cagggctcct cgcccacctc gagacccggg acgggggcct    1380

aggggaccca ggacgtcccc agtgccgtta gcggctttca gggggcccgg agcgcctcgg    1440

ggagggatgg gaccccgggg gcggggaggg ggggcaggct gcgctcaccg cgccttggca    1500

tcctcccccg ggctccagca aacttttctt tgttcgctgc agtgccgccc tacaccgtgg    1560

tctatttccc agttcgaggt aggagcatgt gtctggcagg gaagggaggc aggggctggg 1620
gctgcagccc acagcccctc gcccacccgg agagatccga acccccttat ccctccgtcg 1680
tgtggctttt accccgggcc tccttcctgt tccccgcctc tcccgccatg cctgctcccc 1740
gccccagtgt tgtgtgaaat cttcggagga acctgtttac ctgttccctc cctgcactcc 1800
tgacccctcc ccgggttgct gcgaggcgga gtcggcccgg tccccacatc tcgtacttct 1860
ccctccccgc aggccgctgc gcggccctgc gcatgctgct ggcagatcag ggccagagct 1920
ggaaggagga ggtggtgacc gtggagacgt ggcaggaggg ctcactcaaa gcctcctgcg 1980
taagtgacca tgcccgggca aggggagggg gtgctgggcc ttagggggct gtgactagga 2040
tcgggggacg cccaagctca gtgcccctcc ctgagccatg cctcccccaa cagctatacg 2100
ggcagctccc caagttccag gacggagacc tcaccctgta ccagtccaat accatcctgc 2160
gtcacctggg ccgcaccctt ggtgagtctt gaacctccaa gtccagggca ggcatgggca 2220
agcctctgcc cccggagccc ttttgtttaa atcagctgcc ccgcagccct ctggagtgga 2280
ggaaactgag acccactgag gttacgtagt ttgcccaagg tcaagcctgg gtgcctgcaa 2340
tccttgccct gtgccaggct gcctcccagg tgtcaggtga gctctgagca cctgctgtgt 2400
ggcagtctct catccttcca cgcacatcct cttcccctcc tcccaggctg gggctcacag 2460
acagccccct ggttggccca tccccagtga ctgtgtgttg atcaggcgcc cagtcacgcg 2520
gcctgctccc ctccacccaa ccccagggct ctatgggaag gaccagcagg aggcagccct 2580
ggtggacatg gtgaatgacg gcgtggagga cctccgctgc aaatacatct ccctcatcta 2640
caccaactat gtgagcatct gcaccagggt tgggcactgg gggctgaaca aagaaagggg 2700
cttcttgtgc cctcaccccc cttacccctc aggtggcttg ggctgacccc ttcttgggtc 2760
agggtgcagg ggctgggtca gctctgggcc aggggcccag gggcctggga caagacacaa 2820
cctgcaccct tattgcctgg gacatcaacc agccaagtaa cgggtcatgg gggcgagtgc 2880
aaggacagag acctccagca actggtggtt tctgatctcc tggggtggcg agggcttcct 2940
ggagtagcca gaggtggagg aggatttgtc gccagtttct ggatggaggt gctggcactt 3000
ttagctgagg aaaatatgca gacacagagc acatttgggg acctgggacc agttcagcag 3060
aggcagcgtg tgtgcgcgtg cgtgtgcgtg tgtgtgcgtg tgtgtgtgta cgcttgcatt 3120
tgtgtcgggt gggtaaggag atagagatgg gcgggcagta ggcccaggtc ccgaaggcct 3180
tgaacccact ggtttggagt ctcctaaggg caatgggggc cattgagaag tctgaacagg 3240
gctgtgtctg aatgtgaggt ctagaaggat cctccagaga agccagctct aaagcttttg 3300
caatcatctg gtgagagaac ccagcaagga tggacaggca gaatggaata gagatgagtt 3360

ggcagctgaa gtggacagga tttggtacta gcctggttgt ggggagcaag cagaggagaa 3420

tctgggactc tggtgtctgg cctggggcag acgggggtgt ctcaggggct gggagggatg 3480

agagtaggat gatacatggt ggtgtctggc aggaggcggg caaggatgac tatgtgaagg 3540

cactgcccgg gcaactgaag ccttttgaga ccctgctgtc ccagaaccag ggaggcaaga 3600

ccttcattgt gggagaccag gtgagcatct ggccccatgc tgttccttcc tcgccaccct 3660

ctgcttccag atggacacag gtgtgagcca tttgtttagc aaagcagagc agacctaggg 3720

gatgggctta ggccctctgc ccccaattcc tccagcctgc tcccgctggc tgagtcccta 3780

gcccccctgc cctgcagatc tccttcgctg actacaacct gctggacttg ctgctgatcc 3840

atgaggtcct agcccctggc tgcctggatg cgttccccct gctctcagca tatgtggggc 3900

gcctcagtgc ccggcccaag ctcaaggcct tcctggcctc ccctgagtac gtgaacctcc 3960

ccatcaatgg caacgggaaa cagtgagggt tggggggact ctgagcggga ggcagagttt 4020

gccttccttt ctccaggacc aataaaattt ctaagagagc tactatgagc actgtgtttc 4080

ctgggacggg gcttaggggt tctcagcctc gaggtcggtg ggagggcaga gcagaggact 4140

agaaaacagc tcctccagca cagtcagtgg cttcctggag ccctcagcct ggctgtgttt 4200

actgaacctc acaaactaga agaggaagaa aaaaaaagag agagagaaac aaagagaaat 4260